# EUROPEAN PATENT APPLICATION

(11) **EP 1 803 800 A1**
(43) Date of publication of application: **04.07.2007**
(21) Application number: 05785623.9
(22) Date of filing: 16.09.2005
(51) Int. Cl.: C11D 1/28, C11D 1/12, C11D 1/37

(54) **DETERGENT COMPOSITION**

(30) Priority: 17.09.2004 JP 2004271972; 17.09.2004 JP 2004271973; 15.12.2004 JP 2004362807
(71) Applicant: NOF CORPORATION, Tokyo 150-6019 (JP)
(72) Inventor: SHIMADA, Masahiko, c/o NOF CORPORATION,, Amagasaki-shi, Hyogo 6600095 (JP); FUJITA, Hiroya, c/o NOF CORPORATION,, Amagasaki-shi, Hyogo 6600095 (JP); IWAMA, Takashi, c/o NOF CORPORATION,, Amagasaki-shi, Hyogo 6600095 (JP)
(74) Representative: Vossius & Partner
(86) International application number: PCT/JP2005/017570
(87) International publication number: WO 2006/030990

(57) **Abstract**

The present invention provides a detergent composition comprising a taurine derivative (a) shown by Formula I and an anionic surfactant (b) at a specific ratio, wherein the anionic surfactant (b) is at least one anionic surfactant selected from the group consisting of an anionic surfactant that has a sulfate group or a sulfonic acid group, an acyl amino acid anionic surfactant, and a fatty acid salt having 8 to 22 carbon atoms. The detergent composition of the present invention has good foamability (including foamability in the presence of oil components) and provides a creamy foam texture. When the detergent is used for washing, it does not produce a slimy feeling during rinsing, and its foam can be rinsed off quickly. After washing (after drying), the composition can provide an excellent washed feeling and does not leave the skin feeling tight or rough.

## Description

### Technical Field

The present invention relates to detergent compositions for the body or hair, such as body soap, hand soap, facial cleansers, and hair shampoo. More specifically, the present invention relates to a detergent composition with good foamability (including foamability in the presence of oil components) and a creamy foam texture. When the composition is used for washing, it does not produce a slimy feeling during rinsing, its foam can be rinsed off quickly, and after washing (after drying), the composition leaves an excellent washed feeling that does not leave the skin feeling tight or rough.

### Background Art

Conventionally, anionic surfactants have been used as a main component in detergent compositions for the body or hair such as body soap, hand soap, facial cleansers, and hair shampoo. In order to improve the feeling after washing, such as the tight feeling or rough feeling that occurs after use, humectants or various types of surfactants (such as amphoteric surfactants or nonionic surfactants) also may be used. Examples of the anionic surfactants include anionic surfactants that have a sulfate group or a sulfonic acid group, acyl amino acid anionic surfactants, and fatty acid salts.

Examples of detergent compositions that contain an anionic surfactant that has a sulfate group or a sulfonic acid group include a detergent composition that contains an alkyl ether sulfate anionic surfactant, an amide amino acid amphoteric surfactant, and an alkanol amide nonionic surfactant (Japanese Patent Publication No. 4-79399), a detergent composition that contains an acyl alkyl taurate anionic surfactant, a betaine aminoacetate amphoteric surfactant, and an alkanol amide nonionic surfactant (Japanese Patent Publication No. 59-42038), a detergent composition that contains an acylisethionate anionic surfactant and a betaine amphoteric surfactant (Japanese Laid-Open Patent Publication No. 60-161498), and a detergent composition that contains an amide ether sulfate anionic surfactant and an alkylimino dicarboxylate amphoteric surfactant (Japanese Laid-Open Patent Publication No. 6-172785).

Examples of detergent compositions that contain acyl amino acid anionic surfactants include a detergent composition that contains an N-acyl glutamate and an amide betaine amphoteric surfactant (Japanese Laid-Open Patent Publication No. 60-42496), a detergent composition that contains an N-acyl amino acid salt and an alkanol amide nonionic surfactant (Japanese Laid-Open Patent Publication No. 2-18081.1), and a detergent composition that contains an N-acyl glutamate surfactant and a polyoxyethylene alkyl ether nonionic surfactant (Japanese Laid-Open Patent Publication No. 1-287017).

However, the detergents made of these compositions had the problem of a coarse or light foam texture, and a creamy foam texture is desired. These detergents also left a slimy feeling after contacting skin.

Examples of detergent compositions that contain fatty acid salts include a detergent composition that contains a fatty acid soap and an alkylene oxide adduct of glycerin or diglycerin (Japanese Laid-Open Patent Publication No. 8-20795), a detergent composition that contains a fatty acid salt and a specific quaternary ammonium salt cationic surfactant (Japanese Laid-Open Patent Publication No. 9-104618), a detergent composition that contains a higher fatty acid, an acylisethionate anionic surfactant, and a chelating agent (Japanese Laid-Open Patent Publication No. 5-17341), and a detergent composition that contains a lauric acid salt and a secondary amide type N-acyl amino acid salt (Japanese Laid-Open Patent Publication No. 5-156284).

However, using a detergent made of any one of these compositions adversely affects the fatty acid salt's natural foamability, the quality of its foam texture, and the speed of the foam elimination. Moreover, a slimy feeling remains during rinsing, and the fresh feeling after washing is diminished. At the current time, the rough feeling on the skin after drying has yet to be eliminated.

Further, particularly in detergent compositions for hair such as shampoo, silicone derivatives such as methyl polysiloxane having a high degree of polymerization are often used to make it easier to run fingers through hair when washing or rinsing hair. Examples of such detergent compositions include a detergent composition that contains an acyl alkyl taurate anionic surfactant and a silicone derivative (Japanese Laid-Open Patent Publication No. 4-296398), and a detergent composition that contains an alkyl ether sulfate anionic surfactant, an acyl glutamate anionic surfactant, a betaine amphoteric surfactant, and methylpolysiloxane (Japanese Laid-Open Patent Publication No. 11-29446).

However, since these detergent compositions for hair contain silicone derivatives, their initial foamability is impaired. Further, detergent compositions that contain an anionic surfactant that has a sulfate group or a sulfonic acid group and an acyl amino acid anionic surfactant have poor foamability in the presence of oil components other than silicone derivatives as well.

It is an object of the present invention to provide a detergent composition with good foamability (including foamability in the presence of oil components) and a creamy foam texture. It is also an object of the present invention to provide a detergent composition with various advantages. Namely, when the composition is used for washing, it does not produce a slimy feeling during rinsing, its foam can be rinsed off quickly, and after washing (after drying), the composition leaves an excellent washed feeling that does not leave the skin feeling tight or rough.

### Disclosure of the Invention

A detergent composition of the present invention comprises a taurine derivative (a) and an anionic surfactant (b), wherein the taurine derivative (a) is expressed by Formula I:

R¹CONR²C₂H₄SO₃M (I)

(wherein R¹CO is an acyl group having 6 to 10 carbon atoms, R² is hydrogen or an alkyl group having 1 to 3 carbon atoms, and M is hydrogen, an alkali metal, an alkaline earth metal, ammonium, substituted ammonium, a group derived from an amino acid or an alkali metal salt thereof, a group derived from taurine or an alkali metal salt thereof, or a group derived from N-methyl taurine or an alkali metal salt thereof); wherein the anionic surfactant is at least one anionic surfactant selected from the group consisting of an anionic surfactant that has a sulfate group or a sulfonic acid group, an acyl amino acid anionic surfactant, and a fatty acid salt having 8 to 22 carbon atoms; and wherein the taurine derivative (a) is contained in the composition in a ratio of 0.05 to 30 wt% and the anionic surfactant (b) is contained in the composition in a ratio of 1 to 50 wt%, and a weight ratio of the taurine derivative (a) to the anionic surfactant (b) is 1/100 to 1/1.

In a preferable embodiment, the anionic surfactant (b) is an anionic surfactant that has a sulfate group or a sulfonic acid group.

In a preferable embodiment, the anionic surfactant (b) is an acyl amino acid anionic surfactant.

In a preferable embodiment, the anionic surfactant (b) is a fatty acid salt having 8 to 22 carbon atoms.

Consequently, the present invention can accomplish the following objectives. The present invention provides a detergent composition with good foamability (including foamability in the presence of oil components) and a creamy foam texture. When the composition is used for washing, it does not provide a slimy feeling during rinsing, its foam can be rinsed off quickly, and after washing (after drying) the composition leaves an excellent washed feeling that does not leave the skin feeling tight or rough.

### Best Mode for Carrying Out the Invention

The detergent composition of the present invention comprises a taurine derivative (a) (hereafter, also may be referred to as the component (a)) and a specific anionic surfactant (b) (hereafter, also may be referred to as the component (b)), and as necessary also includes a solvent (such as water) and additives, which are described below. These components will be described below.

### Taurine Derivative (a)

The taurine derivative (a) used for the detergent composition of the present invention is expressed by Formula I.

R¹CONR²C₂H₄SO₃M (I)

(wherein R¹CO is an acyl group having 6 to 10 carbon atoms, R² is hydrogen or an alkyl group having 1 to 3 carbon atoms, and M is hydrogen, an alkali metal, an alkaline earth metal, ammonium, substituted ammonium, a group derived from an amino acid or an alkali metal salt thereof, a group derived from taurine or an alkali metal salt thereof, or a group derived from N-methyl taurine or an alkali metal salt thereof).

In Formula I, R¹CO is an acyl group having 6 to 10 carbon atoms, and the R¹ has a straight chain structure or a branched chain structure. When the number of carbon atoms is 5 or less, irritation of the skin and hair caused by the resultant detergent containing the taurine derivative (a) increases, and the foam texture becomes coarse. Foamability in the presence of oil components also decreases. When the number of carbon atoms is 11 or more, the foam texture becomes coarse and a slimy feeling is produced during rinsing. Further, the foamability in the presence of oil components becomes poor.

In Formula I, R² is hydrogen or an alkyl group having 1 to 3 carbon atoms, and may be a methyl group, an ethyl group, or a propyl group, for example. When the alkyl group has 4 or more carbon atoms, the foamability (including the foamability in the presence of oil components) becomes poor and the foam texture becomes coarse.

In Formula I, M is hydrogen, an alkali metal, an alkaline earth metal, ammonium, substituted ammonium, a group derived from an amino acid (basic amino acid, acidic amino acid, and neutral amino acid) or an alkali metal salt thereof, a group derived from taurine or an alkali metal salt thereof, or a group derived from N-methyl taurine or an alkali metal salt thereof. Specific examples of M include hydrogen, sodium, potassium, 1/2 calcium, 1/2 magnesium, ammonium, triethanolammonium, a group derived from lysine, arginine, glycine, glutamic acid, sodium glycinate, potassium glycinate, or disodium glutamate, or a group derived from taurine, sodium taurate and potassium taurate, or a group derived from N-methyl taurine or sodium N-methyl taurate.

### Anionic Surfactant (b)

The anionic surfactant (b) used for the detergent composition of the present invention is at least one of an anionic surfactant that has a sulfate group or a sulfonic acid group (b.1), an acyl amino acid anionic surfactant (b.2), and a fatty acid salt having 8 to 22 carbon atoms (b.3). These surfactants will be described below.

### (1) Anionic Surfactant that has a Sulfate Group or a Sulfonic acid Group (b.1)

Examples of the anionic surfactant that has a sulfate group or a sulfonic acid group (b.1) used for the detergent composition of the present invention include alkyl ether sulfate anionic surfactants, amide ether sulfate anionic surfactants, acylisethionate anionic surfactants, and acyl alkyl taurate anionic surfactants.

Alkyl ether sulfate anionic surfactants are obtained by esterifying a polyoxyalkylene alkyl ether that contains an alkyl group and a polyoxyalkylene group derived from an alkylene oxide having 2 to 4 carbon atoms with sulfuric acid, and then adding a compound that can form a counter ion to produce a salt. The above alkyl group has 8 to 22 carbon atoms, may be linear or branched, and may be saturated or unsaturated. The mean number of moles of the alkylene oxide unit of the polyoxyalkylene group is 0.5 to 8 moles. Specific examples of such a surfactant include polyoxyethylene lauryl ether sulfate sodium salt.

Amide ether sulfate anionic surfactants can be obtained by esterifying a polyoxyalkylene monoethanolamide that contains an acyl group and a polyoxyalkylene group derived from an alkylene oxide having 2 to 4 carbon atoms with sulfuric acid, and then adding a compound that can form a counter ion to produce a salt. The acyl group has 8 to 22 carbon atoms, may be linear or branched, and may be saturated or unsaturated. The mean number of moles of the alkylene oxide unit of the polyoxyalkylene group is 0.5 to 8 moles. Specific examples of such a surfactant include sodium polyoxyethylene coconut fatty acid monoethanolamide sulfate.

Acylisethionate anionic surfactants can be obtained by adding a compound that can form a counter ion to isethionic acid having an acyl group to form salt. The acyl group has 8 to 22 carbon atoms, may be linear or branched, and may be saturated or unsaturated. Specific examples of such a surfactant include sodium cocoyl isethionate.

Acyl alkyl taurate anionic surfactants can be obtained by adding a compound that can form a counter ion to an amide conjugate in which the hydrogen of an amino group of N-alkyl taurine is substituted by an acyl group, resulting in a salt. The acyl group has 8 to 22 carbon atoms, may be linear or branched, and may be saturated or unsaturated. Specific examples of such a surfactant include sodium cocoyl methyl taurate and a sodium taurate of cocoyl methyl taurine.

The atom or group that forms the counter ion may be a hydrogen atom, an alkali metal, an alkaline earth metal, ammonium, substituted ammonium, a group derived from an amino acid (basic amino acid, acidic amino acid, or neutral amino acid) or an alkali metal salt thereof, a group derived from taurine or an alkali metal salt thereof, or a group derived from N-methyl taurine or an alkali metal salt thereof. Specific examples thereof include sodium, potassium, 1/2 calcium, 1/2 magnesium, ammonium, triethanolammonium, a group derived from lysine, arginine, sodium glycinate, potassium glycinate, or disodium glutamate, and a group derived from taurine, N-methyl taurine, sodium taurate, potassium taurate, or sodium N-methyl taurate.

### (2) Acyl Amino Acid Anionic Surfactant (b.2)

Examples of the acyl amino acid anionic surfactant (b.2) used for the detergent composition of the present invention include acylglutamate anionic surfactants, acylaspartate anionic surfactants, acylglycinate anionic surfactants, acylsarcosinate anionic surfactants, and acyl-β-alaninate anionic surfactants.

All of these surfactants can be obtained by adding a compound that can form a counter ion to amide compound in which the hydrogen of an amino group of the amino acid is substituted with an acyl group to form a salt. The acyl group has 8 to 22 carbon atoms, may be linear or branched, and may be saturated or unsaturated. Specific examples of such a surfactant include sodium cocoyl glutamate, potassium cocoyl aspartate, potassium cocoyl glycinate, sodium lauroyl sarcosinate, and sodium lauroyl-β-alaninate.

Examples of the counter ion include the illustrative counter ions listed above.

### (3) Fatty Acid Salt (b.3)

The fatty acid salt used for the detergent composition of the present invention is a fatty acid salt having 8 to 22 carbon atoms. The fatty acid part of the fatty acid salt may be linear or branched and may be saturated or unsaturated. When the fatty acid has 7 or less carbon atoms, the foamability of the resultant detergent is poor and the foam texture is coarse. When the fatty acid has 23 or more carbon atoms, the foamability and the ability to rinse off the foam are bad, and after drying, the detergent composition leaves a strong feeling of tightness and roughness on the skin.

The atom or group that forms the counter ion of the fatty acid salt may be an alkali metal, ammonium or substituted ammonium, an alkali metal salt of a basic amino acid, an acidic or a neutral amino acid, or an alkali metal salt of a taurine or an N-methyl taurine. Specific examples include sodium, potassium, ammonium, triethanolammonium, a group derived from lysine, arginine, sodium glycinate, potassium glycinate, or disodium glutamate, and a group derived from sodium taurate, potassium taurate, or sodium N-methyl taurate.

Specific examples of the salt of a fatty acid having 8 to 22 carbon atoms include sodium laurate, potassium laurate, triethanolammonium laurate, lysine laurate, arginine laurate, a salt formed from lauric acid and sodium glycinate, a salt formed from lauric acid and disodium glutamate, a salt formed from lauric acid and sodium taurate, a salt formed from lauric acid and potassium taurate, a salt formed from lauric acid and sodium N-methyl taurate, potassium myristate, potassium palmitate, potassium stearate, potassium oleate, potassium cocoate, triethanolammonium cocoate, lysine cocoate, arginine cocoate, a salt formed from coconut oil fatty acid and sodium glycinate, a salt formed from coconut oil fatty acid and disodium glutamate, a salt formed from coconut oil fatty acid and sodium taurate, a salt formed from coconut oil fatty acid and potassium taurate, and a salt formed from coconut oil fatty acid and sodium N-methyl taurate.

### Additives

The detergent composition of the present invention can contain additives to the extent that the effects of the present invention are not impaired. Additives are those normally used for detergents, and examples thereof include lower alcohols, polyhydric alcohols, hydrocarbon oils, natural oils, synthetic triglycerides, ester oils, waxes, silicone derivatives, amphoteric surfactants, nonionic surfactants, cationic surfactants, water-soluble polymer, organic or inorganic salts, pH adjusting agents, antibacterial agents, chelating agents, antioxidants, ultraviolet absorber, vitamins, natural extracts from animal or plants, dyes, pigments, and fragrances.

### Detergent Composition

The body detergent composition of the present invention comprises a taurine derivative (a) and a specific anionic surfactant (b) at a specific ratio, and if necessary also contains solvent (for example, water) and additives.

The taurine derivative (a) is contained in the detergent composition in a ratio of 0.05 to 30 wt%, preferably 0.1 to 20 wt%. When the content of the taurine derivative (a) is lower than 0.05 wt%, then the foam texture of the detergent that contains the taurine derivative (a) is coarse, the foamability in the presence of oil components become poor, and produces a slimy feeling during rinsing. Further, after drying, the detergent leaves the skin feeling tight and rough. When the content of the taurine derivative (a) is greater than 30 wt%, the foam texture becomes coarse and there is a slimy feeling during rising. The component (a) may be used alone or in combination of two or more.

The anionic surfactant (b) is contained in the detergent composition in a ratio of 1 to 50 wt%, preferably 2 to 40 wt%. When the content of the anionic surfactant (b) is lower than 1 wt%, the foam texture of a detergent that contains the anionic surfactant (b) is coarse and the foamability becomes bad. When the content of the anionic surfactant (b) is higher than 50 wt%, the foam texture is coarse and the foamability in the presence of oil components is poor, and there is a slimy feeling during rinsing. Further, the detergent has is highly irritable and leaves the skin feeling tight and rough after drying. The component (b) may be used alone or in combination of two or more.

The weight ratio of the component (a) to the component (b) (a/b) is 1/100 to 1/1. When an anionic surfactant that has a sulfate group or a sulfonic acid group (b.1) or an acyl amino acid anionic surfactant (b.2) is used as the component (b), the weight ratio of the component (a) to the component (b) (a/b) is preferably 1/50 to 1/2. When a fatty acid salt (b.3) is used as the component (b), then the weight ratio of the component (a) to the component (b) (a/b) is preferably 1/50 to 2/3. When the weight ratio is smaller than 1/100, the foam texture is coarse, the foamability in the presence of oil components becomes poor, and a slimy feeling is given during rinsing. Further, the detergent leaves the skin strong feeling of tightness and roughness after drying. When the weight ratio is larger than 1/1, the foam texture becomes coarse and a slimy feeling is given during rinsing.

### EXAMPLES

Next, the present invention will be described in further detail by way of examples.

### Example 1

Using a taurine derivative (a) and an anionic surfactant that has a sulfate group or a sulfonic acid group (b.1) as the anionic surfactant (b), a liquid body detergent was prepared as follows.

A mixture containing 0.2 wt% of taurine derivative 1 as the component (a), 5 wt% of polyoxyethylene (2 mols) lauryl ether sulfate sodium salt as the component (b), various additives listed as 1 to 6 in Table 1 (hereinafter, these additives in combination are referred to as the common additive component 1; 3.5 wt% total), and 91.3 wt% of purified water was prepared to obtain a liquid body detergent. The taurine derivative 1 is shown by Formula 1, in which R¹CO is a straight chain acyl group having 10 carbon atoms (i.e., n-decanoyl group), R² is a methyl group, and M is sodium.

**Table 1**

| | Additives (Common additive component 1) | Amount (wt%) |
|---|---|---|
| 1 | Hydroxyethyl cellulose | 0.5 |
| 2 | Ethylene glycol distearate | 1.7 |
| 3 | Sodium chloride | 0.5 |
| 4 | Hydroxyethane diphosphonic acid | 0.1 |
| 5 | Disodium ethylenediaminetetraacetate | 0.3 |
| 6 | Fragrance | 0.4 |
| Total | | 3.5 |

The obtained detergent was evaluated in terms of its (1) skin irritability, (2) foamability in the presence of oil components, (3) foam creaminess, (4) feeling after washing, and (5) ability of the foam to be rinsed off, through the following methods.

### (1) Skin Irritability

Using a diluted solution in which 1 g of the obtained detergent was diluted with 99 g of purified water, a closed patch test was conducted on 10 male and 10 female panelists with healthy skin. Twenty four hours after the test was begun, the inner side of the upper arm portion of each panelist was observed and evaluated as follows.
2 points: Erythema and swelling are clearly seen on the skin.
1 point: Some erythema is seen on the skin.
0 points: Absolutely no change is seen on the skin.

The mean score of the 20 panelists was found, and it was determined that the detergent has low skin irritability when the score was less than 0.5 points. The mean scores are shown in Table 2. A mean score less than 0.5 is indicated by an open circle, and a mean score of 0.5 or more is indicated by an X-mark.

| | |
|---|---|
| under 0.5 points: | mild skin irritability |
| 0.5 to less than 1.0 points: | moderate skin irritability |
| 1.0 or more points: | severe skin irritability |

### (2) Foamability in the Presence of Oil Components

First, 1 g of the obtained detergent was diluted with 99 g of purified water. Then, 50 g of the resultant dilute solution was weighed out and 0.5 g of macadamia nut oil was added to this solution. The dilute solution containing macadamia nut oil was agitated with a Millser tester (IFM-100, manufactured by Iwatani) for 5 seconds at 40°C. After the agitation, the mixture was allowed to stand for one minute and then the height of the foam was measured. When the height of the foam was 7 cm or more, it was determined that the detergent has good foamability in the presence of an oil component. Table 2 shows the heights of the foam. In Table 2, a foam height of 7 cm or more is indicated by an open circle, and a foam height of less than 7 cm is indicated by an X mark.

### (3) Foam Creaminess

First, 1 g of the obtained detergent was diluted with 99 g of purified water. Then, 50 g of the resultant dilute solution was weighed out and this was agitated with a Millser tester (IFM-100, manufactured by Iwatani) for 5 seconds at 40°C. After the agitation, the mixture was allowed to stand for one minute. Next, the aqueous solution was discarded, while giving a careful attention of not destroying the foam that had formed, and the weight and the volume of the foam remaining in the cup was measured. The density of the foam was calculated from the weight and the volume, and was evaluated as follows.
20 points: The foam density was 0.04 or more.
15 points: The foam density was 0.03 or more and less than 0.04.
10 points: The foam density was 0.02 or more and less than 0.03.
5 points: The foam density was 0.01 or more and less than 0.02.
0 points: The foam density was less than 0.01.

This same test was performed five times, and the mean score was found and rounded off to the first decimal place. When the score was 15 points or more, the detergent was determined to have a creamy foam. The mean scores are shown in Table 2. A mean score of 15 or more is indicated by an open circle, and a mean score of less than 15 is indicated by an X mark.

### (4) Feeling After Washing

Twenty male and female panelists (ages 20 to 40) used 3 g of the detergent that was obtained to wash their forearm and finger tips, and assessed the feeling after washing as follows.

20 points: Panelist felt no skin tightness after washing, and felt extremely refreshed.

15 points: Panelist felt slight skin tightness after washing, but felt refreshed.

10 points: Panelist felt slight skin tightness after washing, and felt that the refreshing feeling was weak.

5 points: Panelist felt skin tightness after washing, and felt that the refreshing feeling was weak.

0 points: Panelist felt strong skin tightness after washing, and did not feel refreshed.

The mean score of the 20 panelists was found and rounded off to the first decimal place. The detergent was determined to have a good feeling after washing when the score was 15 points or more. The mean scores are shown in Table 2. A mean score of 15 or more is indicated by an open circle, and a mean score of less than 15 is indicated by an X mark.

### (5) Ability of Foam to be Rinsed Off

Twenty male and female panelists (ages 20 to 40) used 3 g of the detergent that was obtained to wash their forearm and finger tips, and evaluated the ability of the foam to be rinsed off with tap water as follows.

20 points: Panelist felt that the foam was rinsed off extremely quickly.

15 points: Panelist felt that the foam was rinsed off quickly.

10 points: Panelist felt that the foam was rinsed off slightly quickly.

5 points: Panelist felt that the foam was rinsed off somewhat slowly.

0 points: Panelist felt that the foam was rinsed off slowly.

The mean score of the 20 panelists was found and rounded off to the first decimal place. The detergent was determined to have good foam rinsing-off ability when the score was 15 points or more. The mean scores are shown in Table 2. A mean score of 15 or more is indicated by an open circle, and a mean score of less than 15 is indicated by an X mark.

These results are shown in Table 2.

### Examples 2 to 5

Liquid body detergents were prepared in the same manner as in Example 1 by mixing the components shown in Table 2 at the ratios shown in Table 2. The taurine derivative 2 is shown by Formula 1, in which R¹CO is a straight chain acyl group having 10 carbon atoms (i.e., n-decanoyl group), R² is a methyl group, and M is sodium taurate.

Like in Example 1, the detergents that were obtained were evaluated in terms of their (1) skin irritability, (2) foamability in the presence of oil components, (3) foam creaminess, (4) feeling after washing, and (5) ability of the foam to be rinsed off. The results are shown in Table 2.

### Comparative Examples 1 to 7

Liquid body detergents were prepared in the same manner as in Example 1 by mixing the components shown in Table 2 at the ratio shown in Table 2.

Like in Example 1, the detergents that were obtained were evaluated in terms of their (1) skin irritability, (2) foamability in the presence of oil components, (3) foam creaminess, (4) feeling after washing, and (5) ability of the foam to be rinsed off. The results are shown in Table 2.

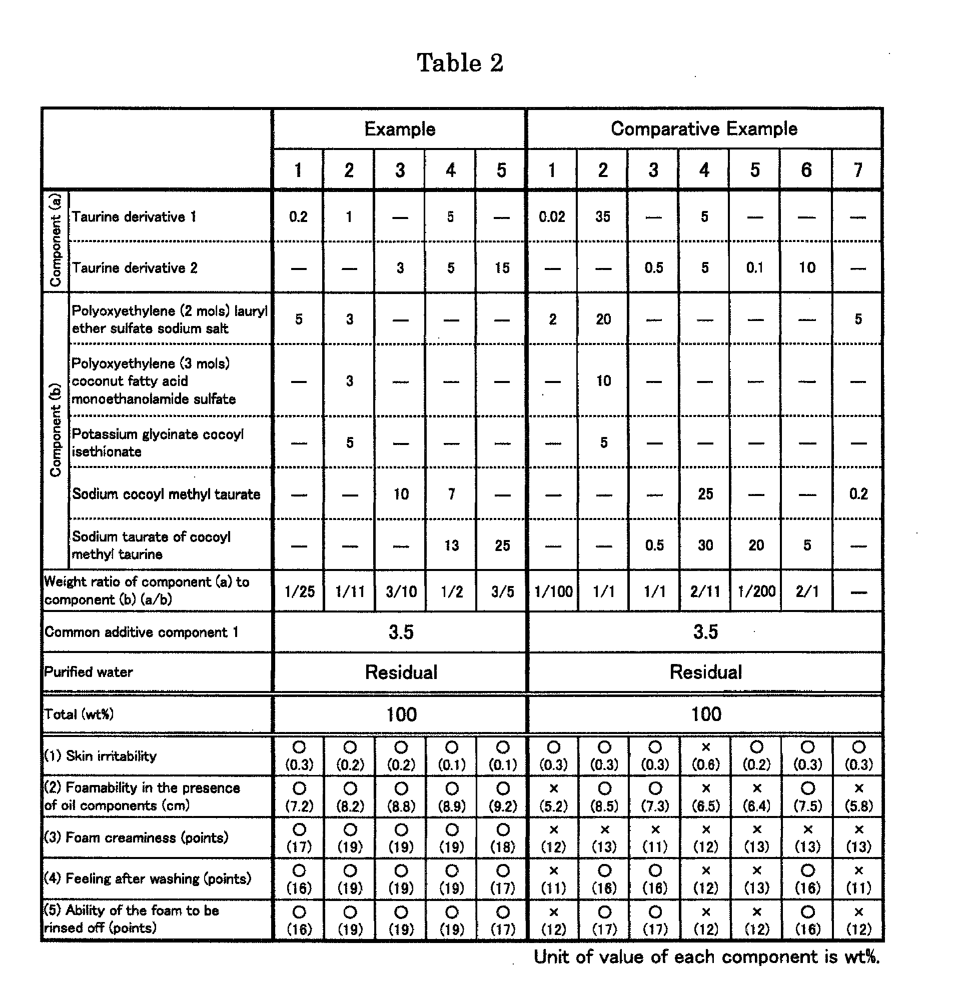

As shown in Table 2, all of the detergents of the present invention obtained in Examples 1 to 5 are excellent detergents with low skin irritability and a good foamability in the presence of oil components. The detergents provide a good feeling when washing is finished, and provide a creamy foam texture. Furthermore, the detergents do not give a slimy feeling during rinsing.

On the other hand, the detergents obtained in Comparative Examples 1 to 7 could not provide sufficient performance. In Comparative Example 1, since the content of the component (a) was below the range of the present invention, the foam texture was coarse and the foamability in the presence of oil components, the feeling after washing, and the ability to rinse off the foam were poor. In Comparative Example 2, since the content of the component (a) was above the range of the present invention, the foam texture was coarse. In Comparative Example 3, since the content of the component (b) was below the range of the present invention, the foam texture was coarse. In Comparative Example 4, since the content of the component (b) was above the range of the present invention, the skin was given strong irritability, and the foamability in the presence of oil components, the feeling after washing, and the ability to rinse off the foam were poor. Furthermore, the foam texture was coarse. In Comparative Example 5, since the weight ratio of the component (a) to the component (b) (a/b) was below the range of the present invention, the foam texture was coarse and the foamability in the presence of oil components, the feeling after washing, and the ability to rinse off the foam were poor. In Comparative Example 6, since the weight ratio of the component (a) to the component (b) (a/b) was above the range of the present invention, the foam texture was coarse. In Comparative Example 7, since the component (a) was not contained in the detergent, the foam texture was coarse, and the foamability in the presence of oil components, the feeling after washing, and the ability to rinse off the foam were poor.

### Example 6

Using a taurine derivative (a) and an acyl amino acid anionic surfactant (b. 2) as the anionic surfactant (b), a liquid body washing agent was prepared as follows.

A mixture containing 0.2 wt% taurine derivative 1 as the component (a), 5 wt% of sodium cocoyl glutamate as the component (b), 3.5 wt% of the common additive components listed in Table 1, and 91.3 wt% of purified water was prepared to obtain a liquid body detergent.

Like in Example 1, the detergent that was obtained was evaluated in terms of its (1) skin irritability, (2) foamability in the presence of oil components, (3) foam creaminess, (4) feeling after washing, and (5) ability of the foam to be rinsed off. The results are shown in Table 3.

### Examples 7 to 10

Liquid body detergents were prepared in the same manner as in Example 6 by mixing the components shown in Table 3 at the ratios shown in Table 3.

Like in Example 1, the detergents that were obtained were evaluated in terms of their (1) skin irritability, (2) foamability in the presence of oil components, (3) foam creaminess, (4) feeling after washing, and (5) ability of the foam to be rinsed off. The results are shown in Table 3.

### Comparative Examples 8 to 14

Liquid body detergents were prepared in the same manner as in Example 6 by mixing the components shown in Table 3 at the ratios shown in Table 3.

Like in Example 1, the detergents that were obtained were evaluated in terms of their (1) skin irritability, (2) foamability in the presence of oil components, (3) foam creaminess, (4) feeling after washing, and (5) ability of the foam to be rinsed off. The results are shown in Table 3.

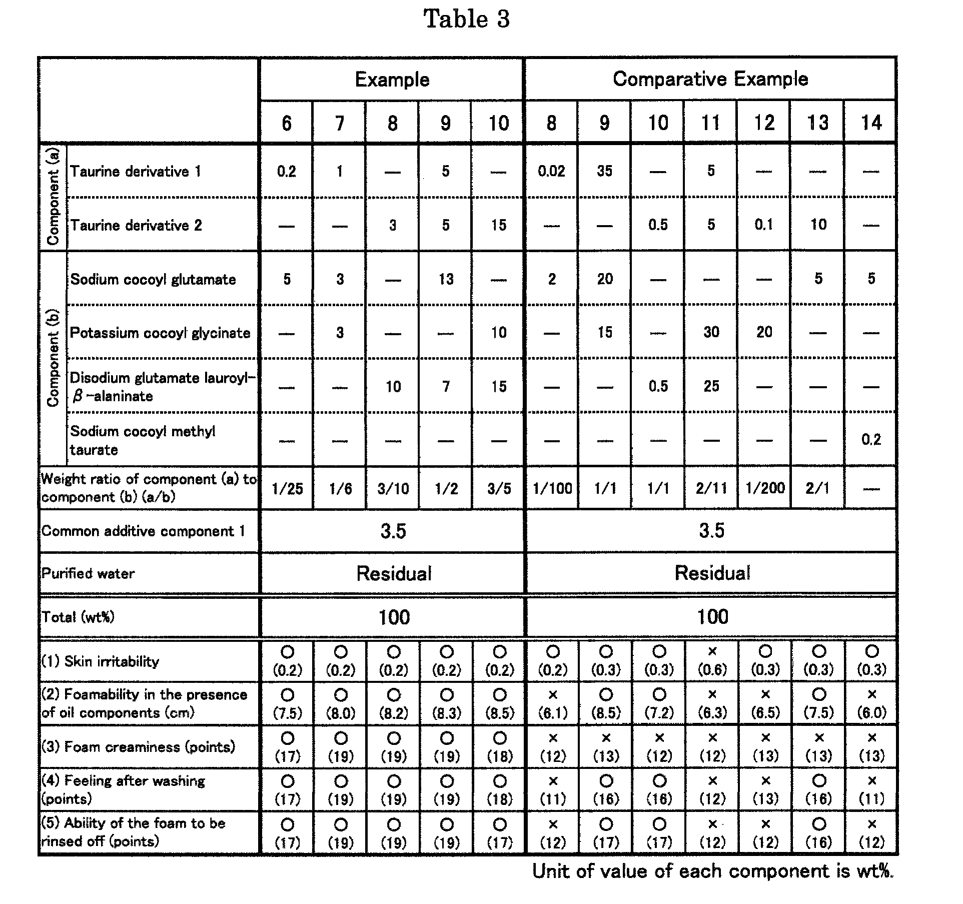

As shown in Table 3, all of the detergents of the present invention obtained in Examples 6 to 10 are excellent detergents with low skin irritability and a good foamability in the presence of oil components. The detergents provide a good feeling when washing is finished, provide a creamy foam texture, and do not produce a slimy feeling during rinsing.

On the other hand, the detergents obtained in Comparative Examples 8 to 14 could not provide sufficient performance. In Comparative Example 8, since the content of the component (a) was below the range of the present invention, the foam texture was coarse and the foamability in the presence of oil components, the feeling after washing, and the ability to rinse off the foam were poor. In Comparative Example 9, since the content of the component (a) was above the range of the present invention, the foam texture was coarse. In Comparative Example 10, since the content of the component (b) was below the range of the present invention, the foam texture was coarse. In Comparative Example 11, since the content of the component (b) was above the range of the present invention, there was strong skin irritability, and the foamability in the presence of oil components, the feeling after washing, and the ability to rinse off the foam were poor. Furthermore, the foam texture was coarse. In Comparative Example 12, since the weight ratio of the component (a) to the component (b) (a/b) was below the range of the present invention, the foam texture was coarse and the foamability in the presence of oil components, the feeling after washing, and the ability to rinse off the foam were poor. In Comparative Example 13, since the weight ratio of the component (a) to the component (b) (a/b) was above the range of the present invention, the foam texture was coarse. In Comparative Example 14, since the component (a) was not contained in the detergent, the foam texture was coarse, and the foamability in the presence of oil components, the feeling after washing, and the ability to rinse off the foam were poor.

### Example 11

Using a taurine derivative (a) and a fatty acid salt (b. 3) as the anionic surfactant (b), a liquid body detergent was prepared as follows.

A mixture containing 0.2 wt% of taurine derivative 1 as the component (a), 5 wt% of potassium laurate as the component (b), 3.5 wt% of the common additive components listed in Table 1, and 91.3 wt% of purified water was prepared to obtaine a liquid body detergent.

The detergent that was obtained was evaluated in terms of its (3) foam creaminess and (5) ability of the foam to be rinsed off. Furthermore, the obtained detergent was evaluated in terms of its (6) foamability, (7) slimy feeling during rinsing, (8) skin tightness after drying, and (9) skin roughness after drying, through the following methods.

### (6) Foamability

First, 1 g of the obtained detergent was diluted with 99 g of purified water. Next, 50 g of the resultant dilute solution was weighed out and agitated with a Millser tester (1FM-100, manufactured by Iwatani) for 5 seconds at 40°C. After the agitation, the mixture was allowed to stand for one minute, and then the height of the foam was measured. When the height of the foam was 7 cm or more, it was determined that the detergent has good foamability. Table 4 shows the height of the foam. A foam height of 7 cm or more is indicated by an open circle, and a foam height of less than 7 cm is indicated by an X mark.

### (7) Slimy Feeling During Rinsing

Twenty male and female panelists (ages 20 to 40) used 3 g of the detergent that was obtained to wash their forearm and finger tips, and assessed the feeling during the detergent was rinsed off as follows.

20 points: Panelist did not experience a slimy feeling, and felt very refreshed.

15 points: Panelist experienced a slightly slimy feeling, but felt refreshed.

10 points: Panelist experienced a slimy feeling, and did not feel very refreshed.

5 points Panelist experienced a clearly slimy feeling, and did not feel refreshed.

0 points: Panelist experienced a persistently slimy feeling, and did not feel refreshed at all.

The mean score of the 20 panelists was found and rounded off to the first decimal place. The detergent was determined that it did not provide a slimy feeling when the score was 15 points or more. The mean scores are shown in Table 4. A mean score of 15 or more is indicated by an open circle, and a mean score of less than 15 is indicated by an X mark.

### (8) Skin Tightness After Drying

Twenty male and female panelists (ages 20 to 40) used 3 g of the detergent that was obtained to wash their forearm and finger tips, and evaluated their skin tightness after drying as follows.

20 points: Panelist did not experience skin tightness after drying.

15 points: Panelist experienced slight skin tightness after drying, but this was not bothersome.

10 points: Panelist experienced slight skin tightness after drying, and this was slightly bothersome.

5 points: Panelist experienced skin tightness after drying.

0 points: Panelist experienced pronounced skin tightness after drying.

The mean score of the 20 panelists was found and rounded off to the first decimal place. The detergent was determined that it did not cause tightness when the score was 15 points or more. The mean scores are shown in Table 4. A mean score of 15 or more is indicated by an open circle, and a mean score of less than 15 is indicated by an X mark.

### (9) Skin Roughness After Drying

Twenty male and female panelists (ages 20 to 40) used 3 g of the detergent that was obtained to wash their forearm and finger tips, and evaluated their skin roughness after drying as follows.

20 points: Panelist did not experience skin roughness after drying.

15 points: Panelist experienced slight skin roughness after drying, but this was not bothersome.

10 points: Panelist experienced slight skin roughness after drying, and this was slightly bothersome.

5 points: Panelist experienced skin roughness after drying.

0 points: Panelist experienced pronounced skin roughness after drying.

The mean score of the 20 panelists was found and rounded off to the first decimal place. The detergent was determined that it did not cause roughness when the score was 15 points or more. The mean scores are shown in Table 4. A mean score of 15 or more is indicated by an open circle, and a mean score of less than 15 is indicated by an X mark.

These results are shown in Table 4.

### Examples 12 to 15

Liquid body detergents were prepared in the same manner as in Example 11 by mixing the components shown in Table 4 at the ratios shown in Table 4.

Like in Example 11, the detergents that were obtained were evaluated in terms of their (3) foam creaminess, (5) ability of the foam to be rinsed off, (6) foamability, (7) slimy feeling during rinsing, (8) skin tightness after drying, and (9) skin roughness after drying. The results are shown in Table 4.

### Comparative Examples 15 to 21

Liquid body detergents were prepared in the same manner as in Example 11 by mixing the components shown in Table 4 at the ratios shown in Table 4.

Like in Example 11, the detergents that were obtained were evaluated in terms of their (3) foam creaminess, (5) ability of the foam to be rinsed off, (6) foamability, (7) slimy feeling during rinsing, (8) skin tightness after drying, and (9) skin roughness after drying. The results are shown in Table 4.

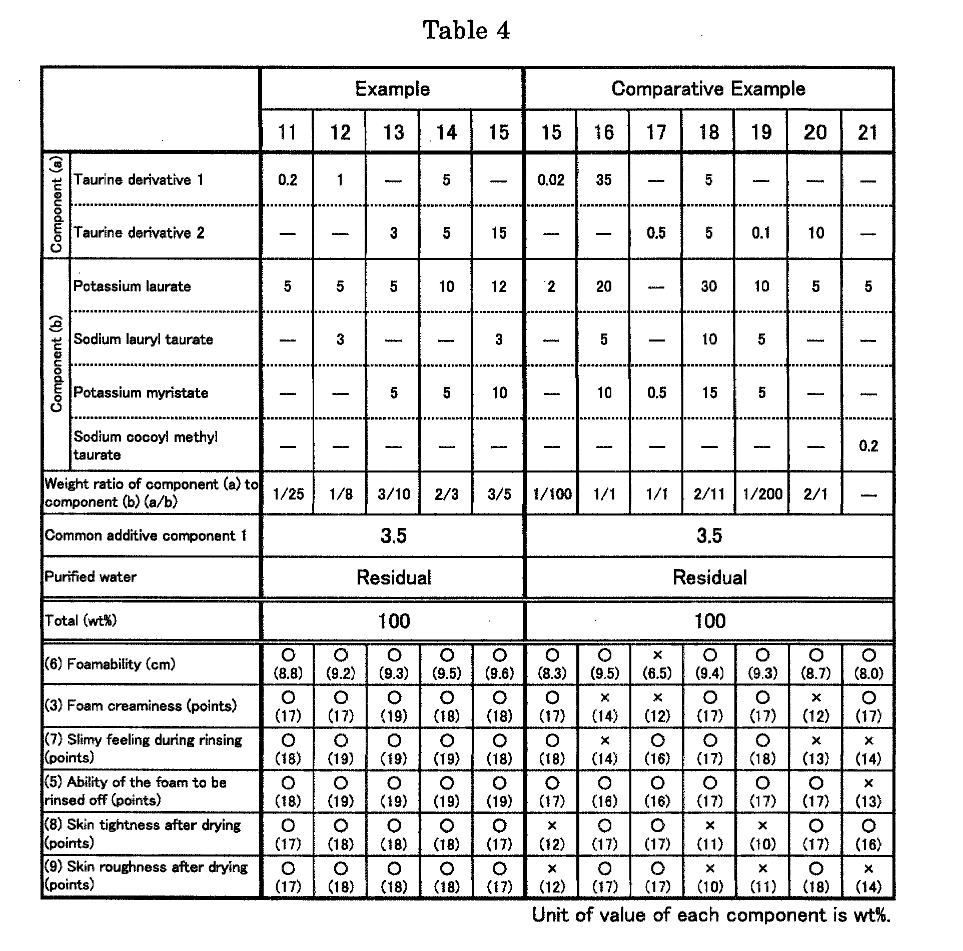

As shown in Table 4, all of the detergents of the present invention obtained in Examples 11 to 15 are excellent detergents with good foamability and a creamy foam texture. The detergents do not produce a slimy feeling during rinsing, their foam is rinsed off quickly, and do not leave skin tightness or roughness after drying.

On the other hand, the detergents obtained in Comparative Examples 15 to 21 could not provide sufficient performance. In Comparative Example 15, since the content of the component (a) was below the range of the present invention, there was a strong feeling of skin tightness and roughness after drying. In Comparative Example 16, since the content of the component (a) was above the range of the present invention, the foam texture was coarse and a slimy feeling was given during rinsing. In Comparative Example 17, since the content of the component (b) was below the range of the present invention, the foamability was poor and the foam texture was coarse. In Comparative Example 18, the content of the component (b) was above the range of the present invention, there was a strong feeling of skin tightness and roughness after drying. In Comparative Example 19, since the weight ratio of the component (a) to the component (b) (a/b) was below the range of the present invention, there was a strong feeling of tightness and roughness in the skin after drying. In Comparative Example 20, since the weight ratio of the component (a) to the component (b) (a/b) was above the range of the present invention, the foam texture was coarse and a slimy feeling was given during rinsing. In Comparative Example 21, since the component (a) was not contained in the detergent, a slimy feeling was given during rinsing, and the ability to rinse off the foam was poor. Furthermore, after drying, a rough feeling was left on the skin.

### Example 16

A mixture containing 5 wt% of taurine derivative 1 as the component (a), 7 wt% of sodium cocoyl glutamate as the component (b), 5.9 wt% of the various additives (common additive component 2) listed as 1 to 9 in Table 5, and 82.1 wt% of purified water was prepared to obtain a hair shampoo.

**Table 5**

| | Additives (Common additive component 2) | Amount (wt%) |
|---|---|---|
| 1 | O-[2-hydroxy-3-(trimethylammonio)propyl] hydroxyethyl cellulose chloride | 0.7 |
| 2 | Coconut fatty acid monoethanolamide | 0.5 |
| 3 | Ethylene glycol distearate | 1.7 |
| 4 | Dimethylpolysiloxane emulsion^{*1} | 2 |
| 5 | Citric acid | 0.2 |
| 6 | Trisodium citrate | 0.2 |
| 7 | Methylparaben | 0.2 |
| 8 | Propylparaben | 0.1 |
| 9 | Sodium salicylate | 0.3 |
| Total | | 5.9 |

| | | |
|---|---|---|
| *1: KM-902 manufactured by Shin-Etsu Chemical Co.,Ltd. | | |

The hair shampoo that was obtained was evaluated in terms of its (10) foamability, (11) foam creaminess, (12) feeling after washing, and (13) ability of the foam to be rinsed off, through the following methods.

### (10) Foamability

Twenty male and female panelists (ages 20 to 40) used 5 g of the hair shampoo that was obtained and washed their hair, and evaluated the foamability at that time as follows.
20 points: Panelist felt that the foamability was very good.
15 points: Panelist felt that the foamability was somewhat good.
10 points: Panelist felt that the foamability was normal.
5 points: Panelist felt that the foamability was somewhat poor.
0 points: Panelist felt that the foamability was poor.

The mean score of the 20 panelists was found and rounded off to the first decimal place. The hair shampoo was determined to have good foamability when the score was 15 points or more. The mean scores are shown in Table 6. A mean score of 15 or more is indicated by an open circle, and a mean score of less than 15 is indicated by an X mark.

### (11) Foam Creaminess

First, 1 g of the obtained hair shampoo was diluted with 99 g of purified water. Then, 50 g of the resultant dilute solution was weighed out and this was agitated with a Millser tester (IFM-100, manufactured by Iwatani) for 5 seconds at 40°C. After the agitation, the mixture was allowed to stand for one minute. Next, the aqueous solution was discarded, while giving a careful attention of not destroying the foam that had formed, and the weight and the volume of the foam remaining in the cup was measured. The density of the foam was calculated from the weight and the volume, and was evaluated as follows.
20 points: The foam density was 0.04 or more.
15 points: The foam density was 0.03 or more and less than 0.04.
10 points: The foam density was 0.02 or more and less than 0.03.
5 points: The foam density was 0.01 or more and less than 0.02.
0 points: The foam density was less than 0.01.

This same test was performed five times, and the mean score was found and rounded off to the first decimal place. The hair shampoo was determined to have a creamy foam when the score was 15 points or more. The mean scores are shown in Table 6. A mean score of 15 or more is indicated by an open circle, and a mean score of less than 15 is indicated by an X mark.

### (12) Feeling After Washing

Twenty male and female panelists (ages 20 to 40) used 5 g of the hair shampoo that was obtained to wash their head hair and scalp, and assessed the feeling after washing as follows.

20 points: Panelist felt extremely refreshed after washing.

15 points: Panelist felt refreshed after washing.

10 points: After washing, panelist felt that hair smoothness was not obtained sufficiently and that slightly refreshed.

5 points: After washing, panelist felt that hair smoothness was insufficient and that slightly refreshed.

0 points: After washing, panelist felt that hair smoothness was not obtained, and did not feel refreshed.

The mean score of the 20 panelists was found and rounded off to the first decimal place. The hair shampoo was determined to have a good feeling after washing when the score was 15 points or more. The mean scores are shown in Table 6. A mean score of 15 or more is indicated by an open circle, and a mean score of less than 15 is indicated by an X mark.

### (13) Ability of Foam to be Rinsed Off

Twenty male and female panelists (ages 20 to 40) used 5 g of the hair shampoo that was obtained to wash their head hair and scalp, and evaluated the ability of foam to rinse off with tap water as follows.

20 points: Panelist felt that the foam was rinsed off extremely quickly.

15 points: Panelist felt that the foam was rinsed off quickly.

10 points: Panelist felt that the foam was rinsed off slightly quickly.

5 points: Panelist felt that the foam was rinsed off somewhat slowly.

0 points: Panelist felt that the foam was rinsed off slowly.

The mean score of the 20 panelists was found and rounded off to the first decimal place. When the score was 15 points or more, the hair shampoo was determined to have foam that can be rinsed off easily. The mean scores are shown in Table 2. A mean score of 15 or more is indicated by an open circle, and a mean score of less than 15 is indicated by an X mark.

These results are shown in Table 6.

### Example 17

A hair shampoo was prepared in the same manner as in Example 16 by admixing the components shown in Table 6 at the ratios shown in Table 6.

Like in Example 16, the hair shampoo that was obtained was evaluated in terms of its (10) foamability, (11) foam creaminess, (12) feeling after washing, and (13) ability of the foam to be rinsed off. The results are shown in Table 6.

### Comparative Examples 22 to 25

Hair shampoos were prepared in the same manner as in Example 16 by admixing the components shown in Table 6 at the ratios shown in Table 6.

Like in Example 16, the hair shampoos that were obtained were evaluated in terms of their (10) foamability, (11) foam creaminess, (12) feeling after washing, and (13) ability of the foam to be rinsed off. The results are shown in Table 6.

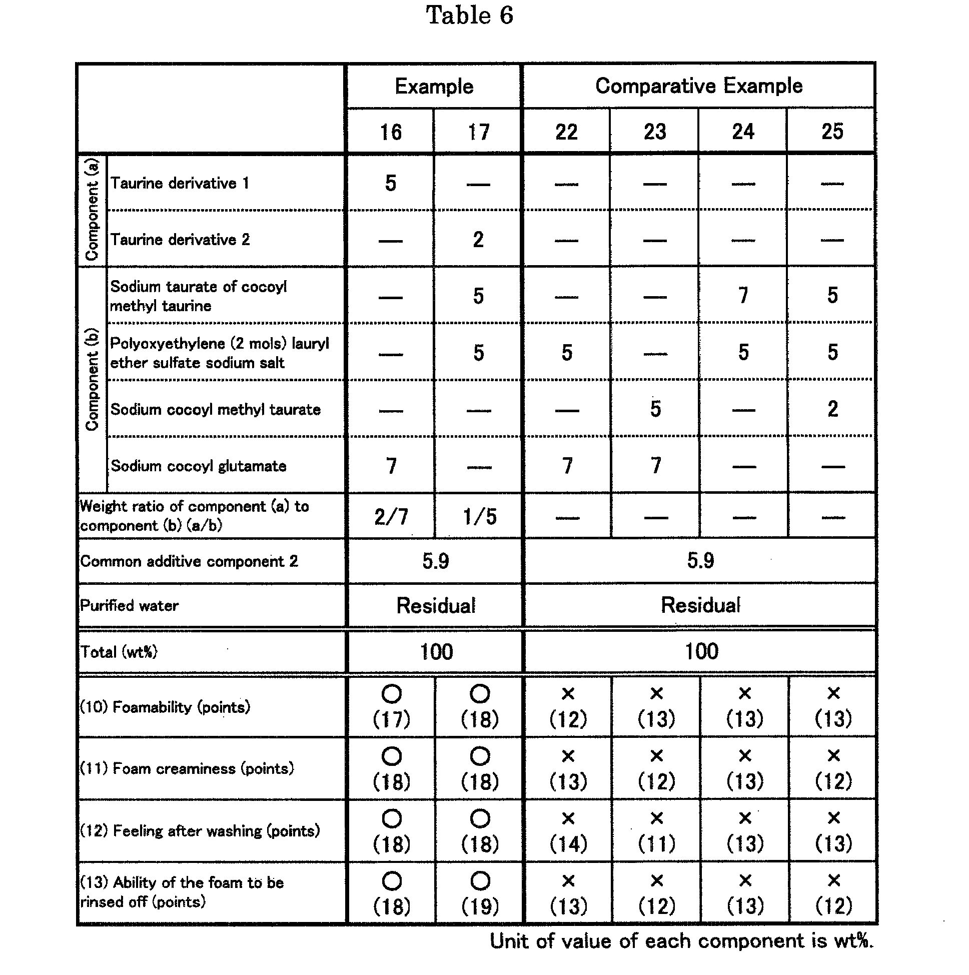

As shown in Table 6, both of the detergents (hair shampoos) of the present invention obtained in Examples 16 and 17 are excellent detergents with good foamability and a creamy foam texture. The detergents do not produce a slimy feeling during rinsing, their foam is rinsed off quickly, and after drying, do not cause the skin to feel tight or rough.

On the other hand, since none of the hair shampoos obtained in Comparative Examples 22 to 25 contain the component (a), the foam texture was rough, and the foamability, the feeling after washing, and the ability to rinse away the foam were poor.

### Industrial Applicability

The detergent composition of the present invention has good foamability (including foamability in the presence of oil components) and provides a creamy foam texture. When the detergent is used for washing, it does not produce a slimy feeling during rinsing, and its foam can be rinsed off quickly. After washing (after drying), the composition can provide an excellent washed feeling and does not leave the skin feeling tight or rough. Thus, the detergent composition of the present invention can be used for body soap, hand soap, facial cleansers, and hair shampoo, and the like.

## Claims

1. A detergent composition comprising a taurine derivative (a) and an anionic surfactant (b),
wherein the taurine derivative (a) is expressed by Formula I:
R¹CONR²C₂H₄SO₃M (I)
(wherein R¹CO is an acyl group having 6 to 10 carbon atoms, R² is hydrogen or an alkyl group having 1 to 3 carbon atoms, and M is hydrogen, an alkali metal, an alkaline earth metal, ammonium, substituted ammonium, a group derived from an amino acid or an alkali metal salt thereof, a group derived from taurine or an alkali metal salt thereof, or a group derived from N-methyl taurine or an alkali metal salt thereof);
wherein the anionic surfactant is at least one anionic surfactant selected from the group consisting of an anionic surfactant that has a sulfate group or a sulfonic acid group, an acyl amino acid anionic surfactant, and a fatty acid salt having 8 to 22 carbon atoms; and
wherein the taurine derivative (a) is contained in the composition in a ratio of 0.05 to 30 wt% and the anionic surfactant (b) is contained in the composition in a ratio of 1 to 50 wt%, and a weight ratio of the taurine derivative (a) to the anionic surfactant (b) is 1/100 to 1/1.

2. The detergent composition of claim 1, wherein the anionic surfactant (b) is an anionic surfactant that has a sulfate group or a sulfonic acid group.

3. The detergent composition of claim 1, wherein the anionic surfactant (b) is an acyl amino acid anionic surfactant.

4. The detergent composition of claim 1, wherein the anionic surfactant (b) is a fatty acid salt having 8 to 22 carbon atoms.
